(19) Europäisches Patentamt
European Patent Office
Office européen des brevets

(11) Publication number: **0 049 072 B1**

(12) **EUROPEAN PATENT SPECIFICATION**

(45) Date of publication of patent specification: **31.01.90**

(51) Int. Cl.[5]: **C 07 D 473/18,** A 61 K 31/505

(21) Application number: **81304227.2**

(22) Date of filing: **15.09.81**

(60) **Divisional application 87104286 filed on 24.03.87.**

(54) **Guanine compounds and their use for the preparation of anti-viral agents.**

(30) Priority: **16.09.80 US 187631**

(43) Date of publication of application:
**07.04.82 Bulletin 82/14**

(45) Publication of the grant of the patent:
**31.01.90 Bulletin 90/05**

(84) Designated Contracting States:
**AT BE CH DE FR GB IT LI LU NL SE**

(56) References cited:
**EP-A-0 066 208**
**EP-A-0 072 027**
**EP-A-0 074 306**
**GB-A-1 523 865**
**US-A-4 146 715**

**TETRAHEDRON LETTERS, vol. 21, 1980, Pergamon Press Ltd. OXFORD (GB); K.K. OGILVIE et al.:"Ring open analogues of deoxynucleotides" pages 327-330**

(73) Proprietor: **SYNTEX (U.S.A.) INC.**
**3401 Hillview Avenue P.O. Box 10850**
**Palo Alto California 94303 (US)**

(72) Inventor: **Ogilvie, Kelvin K.**
**54 Place de Bretagne**
**Candiac Province of Quebec (CA)**

(74) Representative: **Perry, Robert Edward et al**
**GILL JENNINGS & EVERY 53-64 Chancery Lane**
**London WC2A 1HN (GB)**

(58) References cited:

**CHEMICAL ABSTRACTS, vol. 84, 1976, page 605, abstract no. 180515n COLUMBUS, OHIO (US)**
**Alfred Burger: Medicinal Chemistry (3), part I, 74-76**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 049 072 B1

**Description**

This invention relates to guanine compounds and to the use of such compounds as anti-viral agents.

Nucleosides comprise a D-ribose or 2-deoxy-D-ribose sugar unit, chemically bonded to a purine or pyrimidine base selected from adenine, cytosine, guanine, thymine and uracil, *via* a nuclear nitrogen atom of the base. Since they are units of nucleic acids founds naturally in living cells, it has been speculated previously that nucleosides and nucleotides and their analogues might have potential as chemotherapeutic agents. Any practical value they may have, however, is often greatly reduced by their ready deamination *in vivo* by deaminases. Studies have been conducted to determine the relationship between structure and activity for both substrates and inhibitors of adenosine deaminase, some such studies involving ring-opened analogues of nucleosides.

To date, however, despite several promising reports of novel compounds, no such compounds have been produced and developed for chemotherapeutic use, with the possible exception of acycloguanosine which is one of the purine compounds, described in US—A—4146715, having the formula

$$Z—CHR^6—X—CHR^3—CHR^4R^5$$

wherein Z is a purine group; X is oxygen of sulphur; $R^3$ is H, alkyl, hydroxyalkyl, benzyloxyalkyl or phenyl; $R^4$ is H, hydroxy or alkyl; $R^5$ is H, OH, amino, alkyl, hydroxyalkyl, benzoyloxy, benzoyloxymethyl, benzyloxy, sulphamoyloxy, phosphate, acyloxy or substituted carbamoyl; and $R^6$ is H or alkyl; with the proviso that, when X is oxygen, $R^3$, $R^4$ and $R^6$ are H, and $R^5$ is H, OH or benzyloxy, Z is not adenine or 6-N-methyladenine.

Ogilive *et al,* Tetrahedron Letters, *21* (1980) 327—30, disclose purine and pyrimidine compounds of the formula

$$Z—CH_2—O—CH(CH_2OR)—CH_2OR' \qquad I$$

wherein Z is adenine or thymine and R and R' are chosen from H, monomethoxytrityl, t-butyldimethylsilyl and benzyl.

EP—A—0066208 (filed 19.05.82, priority date 21.05.81) discloses and claims the compound of formula I in which Z is guanine and R and R' are each H, i.e. 9-[[2-hydroxy-1-(hydroxymethyl)ethoxy]methyl]guanine, and its antiviral activity.

Novel guanine derivatives according to the present invention have the formula II

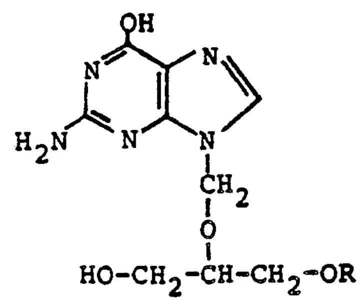

II

wherein R is H, silyl, substituted silyl, $C_{1-6}$ alkyl or phenyl($C_{1-6}$ alkyl). An example of compounds of this invention is the compound (R = H) disclosed and claimed in EP—A—0066208 (see above).

It will be appreciated that the compounds according to the present invention are closely analogous in structure and groupings to naturally-occurring nucelosides and nucleotides. The essential chain arrangements and lengths are maintained. The appropriate O and OH functional groups, which in biological environments actively bind to biological centres, are maintained in their natural sequences and disposition relative to the base, but optionally modified with "protecting" groups. Indeed, the groups adjacent to the bases are so similar in chemical constitution to deoxyribose compounds that they can assume the essential conformation of the deoxyribose ring under appropriate conditions. The fundamental difference is that the compounds of the present invention lack the structural rigidity of carbohydrate rings, which renders them unpredictably different in properties and behaviour. Also, the C—4' positions not chiral, in compounds of formula I or formula II, so that stereoisomers do not arise. Each hydroxyl is primary. There can be no syn-anti isomerism about the glycosidic bond.

Compounds of formula II may be made by coupling the appropriately protected guanine with the appropriate alkyl residue. The synthesis may be initiated by treating 1,3-dichloro-2-propanol with sodium benzylate under a nitrogen atmosphere, followed by s-trioxane and HCl, to prepare the chloromethoxy derivative, care being taken to remove excess water. This derivative of 1,3-dibenzyloxy-2-propanol may be coupled to the appropriately protected base in DMF using triethylamine as acid scavenger. The product may be debenzylated to give a compound of formula II, e.g. with hydrogen over palladium oxide in methanol. Protecting groups, if desired, are put on by standard known methods.

Compounds, of the present invention show antiviral activity, accompanied by low cell toxicity, rendering them potentially useful in therapeutic compositions to combat specific viral invaders of living

mammalian cells. For example, (9-[[2-hydroxy-1-(hydroxymethyl)ethoxy]methyl]guanine is extremely active against herpes simplex virus, and in fact shows an activity of magnitude much greater than that of acycloguanosine, as well as being active against a broader range of viruses than acycloguanosine.

It will of course be understood that the present invention extends to cover pharmaceutically acceptable salts of the compounds described herein.

Compounds according to the present invention may be administered to a patient parenterally, interthecally applied topically as ointment, cream, aerosol or powder, or even on occasion given as eye or nose drops or orally. In general, methods of administration and dosage formulations thereof follow the known, published methods used with known antiviral drugs such as acycloguanosine, adenine arabinoside and 2'-deoxy-5-iodouridine. Effective unit doses for administration of the compositions interthecally or parenterally, calculated as free base, as suitably 0.1—100, preferably 0.5—20 and most preferably about 5, mg per kg mammal body weight, on the basis of a dosage administered 2—4 times per day.

Orally administrable compositions are preferably in fine powder or granule form, with diluting and/or dispersing and/or surface active agents, e.g. in water or in a syrup dispersion, or as tablets or capsules. Solutions of the compounds in distilled water or saline, e.g. isotonic solutions and optionally buffered with phosphate, of concentration 1—20%, preferably 2—15% and most preferably around 10%, are suitable for parenteral or interthecal administration. Ointments (topical or cream) may be compounded for treatment of external infections, e.g. with an oi-in-water cream base, in a concentration of 0.1—10%, preferably up to about 3%, most preferably about 1%, w/v active ingredient. They may be compounded with paraffin oil, with petrolatum to form emulsion optionally with a surfactant for stabilising purposes, or with dimethyl sulfoxide.

The invention is further illustrated by the following non-limitative Example.

## Example

9-[[2-hydroxy-1-(hydroxymethyl)ethoxy]methyl]guanine

2-N-acetylguanine (1.93 g, 10 mmoles) and ammonium sulfate (100 mg) were suspended in HMDS (20 ml). The stirred mixture was refluxed for 3 hours when it became clear. The excess HMDS was removed under reduced pressure on a hot water bath to yield a white solid, silyl-protected 2-N-acetylguanine, which was used without further purification. The white solid was dissolved in DCE (50 ml); 1,3-dibenzyloxy-2-chloromethyoxypropane (5 mmoles) was added followed by freshly distilled anhydrous stannic chloride (1 ml). The solution was allowed to stand overnight at room temperature. The solution was poured into a mixture of aqueous sodium bicarbonate and chloroform and shaken. The mixture was filtered through Celite (registered Trade Mark) to remove the precipitate. The phases were separated and the aqueous phase was extracted once with chloroform.

The combined organic phases were washed with water, dried with anhydrous sodium sulfate, and evaporated under reduced pressure to yield 2.19 g of material. The reaction product was dissolved in chloroform (5 ml) and applied to a tlc silica column (9 × 6.5 cm). The column was first eluted with chloroform (60 ml) and then the solvent was changed to 2% methanol in chloroform. Three reasonably pure fractions were obtained from test tubes 15 (0.047 g), 19—20 (0.148 g) and 23—27 (0.43 g). Each of the samples was crystallised with ethanol, and gave c. 20 mg, 64 mg and 250 mg of material respectively. On the basis of U.V. spectra, the 250 mg material was determined to be 2-N-acetyl-9-[[2-benzyloxy-1-(benzyloxymethyl)ethoxy]methyl]guanine.

This N-actyle compound was recrystallised from ethanol and gave m.p. 142—4°C. The U.V. spectrum gave: λ max (EtOH) 257,281 nm min 227,272, λ max ($H_2O$) 259,278 (shoulder), (pH1) 262, (pH13) 263.

The N-acetyl compound (1.288 g, 0.00270 mole) was dissolved in pyridine (1.5 ml), and concentrated ammonium hydroxide (6 ml) was added. The flask was tightly stoppered and put in a water bath set at 55°C. After 15 hours, crystals had precipitated which were filtered and washed with ethanol. The crystals gave m.p. 170—177°C, were recrystallised from ethanol (60 ml) to yield 0.863 g (0.00191 mole, 70.7%) of 9-[[2-benzyloxyl-1-(benzyloxymethyl)ethoxy]metyl]guanine, m.p. 180—182°C.

This deacetylated compound (0.665 g, 0.00139 mole) was dissolved in refluxing ethanol (40 ml). Palladium oxide (0.67 g) was added followed by cyclohexene (20 ml). Reflux was continued and, after 2 hours, tlc showed that there was still much starting compound present. Therefore, more palladium oxide (0.6 g, Aldrich Gold Label) was added. After 5.5 hours, the reaction still seemed to progress slowly; therefore, palladium black (0.5 g previously stored several months under water and now dried by filtration and washing with ethanol) was added. After seven hours, more cyclohexene (15 ml) was added. After 12 hours, the reaction was not complete according to the tlc but, after 22.5 hours, tlc showed that the reaction was complete.

The reaction mixture was filtered hot and the catalyst was washed with hot 95% ethaaol. Upon cooling, crystals were deposited which were filtered and washed with 95% ethanol. The yield of crystals was 127 mg which did not melt up to 360°C although they had become dark-brown in colour. The catalyst still had product absorbed, and so it was washed with hot 75% ethanol. The washing was combined with the mother liquor from above and evaporated under reduced pressure. The residue was dissolved in a hot mixture of water (2.5 ml) and ethanol (2.5 ml) and then more ethanol (17.5 ml) was added with heating. The solution was allowed to crystallise. The crystals (155 mg) were filtered and washed with ethanol. The crystals did not melt up to 360°C. The mother liquor yielded about 60 mg of residue. Therefore the yield of

product was 282 mg (0.00110 mole, 79%). The UV spectrum gave: $\lambda_{max}$ (EtOH), 254, 270 shoulder, ($H_2O$) 252, 269 (shoulder), (pH 1) 254, 272 (shoulder) (pH 13) 262. This is the title product.

Testing and Evaluation

Herpes simplex virus (HSV) strains were grown and titrated at 36°C in human fetal fibroblasts derived from fetal tissues, and used for virus work before the tenth passage. Cells were grown and maintained in basal medium Eagle (BME; Auto-Pow, Flow Laboratories) supplemented with 0.112% sodium bicarbonate, 2mM 1-glutamine, 2 mg neomycin per 100 ml and 5—20% calf serum. 5% BME, as described hereafter, indicates medium containing 5 ml of calf serum in a total volume of 100 ml.

The titer of the HSV strains is determined by a plaque titration method (Roizman & Roane, "Virology", *15*, 75—79, 1961). Tissue culture dishes are seeded with cells and used for assays when approximately 75% monolayer. Volumes (0.2 ml) of logarithmic dilutions of the strain are inoculated on to each of two tissue culture dishes, and adsorbed for 1 hr. with intermittent shaking; the inoculum is removed, and 2 ml of 5% BME containing 0.5% human immune serum globuline are added. After a 48 hour incubation period at 36°C in a 5% $CO_2$ atmosphere, the overlay medium is removed, and the cell sheets are strained with a 0.05% aqueous crystal violet solution. The number of plaques is counted, the duplicates averaged, and the number of plaque-forming units calculated.

Activity against the herpex simplex strains is tested using a stock solution of 1.2 mg of the freshly-prepared compound of the Example, dissolved in BME. Appropriate dilution in 5% BME containing 0.5% human immune serum globuline is made just before usage.

Tissue culture dishes (35 by 10 mm) with approximately 75% cell monolayer are inoculated with approximately 50 plaque-forming units of HSV per 0.2 ml, and the virus adsorbed for 1 hour, with intermittent shaking. Afer removal of the inoculum, 2 ml of 5% BME with 0.5% immune globulin and three-fold dilutions of the appropriate drug are added. One set of dishes receives no drug and is used as a control. After a 48-hour incubation period at 36°C in a 5% $CO_2$ atmosphere, the overlay medium is removed, the cells are stained as described above, and plaques are counted. The counts of replicate plates are averaged, and the number of plaques emerging in the presence of each drug dilution is calculated. The reduction in plaque size caused by the concentration of the drug, as compared with the relevant control, is also measured, visually. A reduction in plaque number indicates that the added compound is preventing the reproduction of the viral cells. A reduction in the area of the growing plaque indicates inhibition of plaque growth, i.e. inhibition of viral reproduction, caused by the drug.

The results showed that the compound of the Example was outstandingly effective against herpes simplex. It reduced plaque size by 25% at concentration as low as 0.02 µg/ml, by 50% at 0.1 concentration and by 75% at 0.8 concentration. It reduced the plaque number by 25% at 0.04 µg/ml, by 50% at 0.1 concentration and by 75% at 0.2 concentration. At the highest concentration level tested (250 µg/ml), it showed no evidence of toxicity towards the cells. When the compound was used at concentrations of 2 µg/ml and higher, no plaque formation or growth was detected. Significant activity was detected at concentrations as low a 0.007 µg/ml.

Essentially similar results were obtained when the compound was tested against 8 different strains of HSV type I, and 6 different strains of HSV type II.

The compound of the Examples was tested by standard plaque titration methods to determine its activity against various other types of virus, using plaque tests as described above. The compound showed activity against VSV at low concentrations, and against coxsackie virus CVB3 at low concentrations. The compound was also tested against the virus Varicella Zoster, responsible for chicken pox and shingles infections in man, by similar procedures. It was found to be active against Varicella Zoster at concentrations as low as 13 µg/ml.

**Claims for the Contracting States: BE CH DE FR GB IT LI LU NL SE**

1. A guanine derivative of the formula

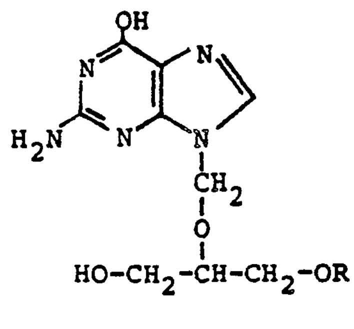

wherein R is H, silyl, substituted silyl, $C_{1-6}$ alkyl or phenyl ($C_{1-6}$ alkyl).

2. Use of a guanine derivative as defined in claim 1, for the manufacture of a medicament for the treatment of viral infections in live mammalian cells.

**Claims for the Contracting State: AT**

1. A process for preparing a guanine derivative of the formula

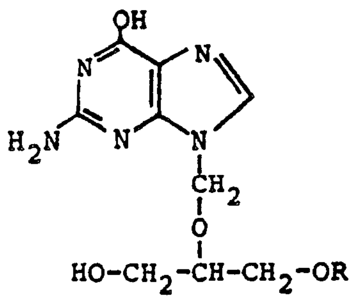

wherein R is H, silyl, substituted silyl, $C_{1-6}$ alkyl or phenyl ($C_{1-6}$ alkyl), which comprises reacting an appropriately protected guanine with an optionally protected 1,3-dihydroxy-2-propoxymethyl halide or other reactive derivative and, as necessary, removing protecting groups and/or introducing a group R other than H.

2. Use of a guanine derivative as defined in claim 1, for the manufacture of a medicament for the treatment of viral infections in live mammalian cells.

**Patentansprüche für die Vertragsstaaten: BE CH DE FR GB IT LI LU NL SE**

1. Guaninderivet der Formel

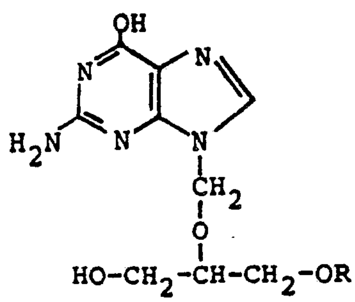

worin R H, Silyl, substituiertes Silyl, $C_{1-6}$-Alkyl oder Phenyl($C_{1-6}$-alkyl) ist.

2. Verwendung eines wie in Anspruch 1 definierten Guaninderivats zur Herstellung eines Medikaments zur Behandlung von Virusinfektionen in lebenden Säugetierzellen.

**Patentansprüche für den Vertragsstaat: AT**

1. Verfahren zur Herstellung eines Guaninderivats der Formel

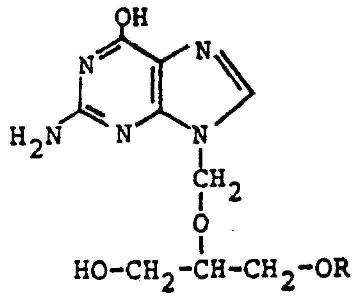

worin R H, Silyl, substituiertes Silyl, $C_{1-6}$-Alkyl oder Phenyl($C_{1-6}$-alkyl) ist, umfassend das Umsetzen eines geeignet geschützten Guanins mit einem gegebenenfalls geschützten 1,3-Dihydroxy-2-proproxymethyl-halogenid oder einem anderen reaktiven Derivat, und, insofern als erforderlich, das Entfernen von Schutzgruppen und/oder das Einführen einer von H verschiedenen Gruppe R.

2. Verwendung eines wie in Anspruch 1 definierten Guaninderivats zur Herstellung eines Medikaments zur Behandlung von Virusinfektionen in lebenden Säugetierzellen.

**Revendications pour les Etats contractants: BE CH DE FR GB IT LI LU NL SE**

1. Dérivé de guanine de formule

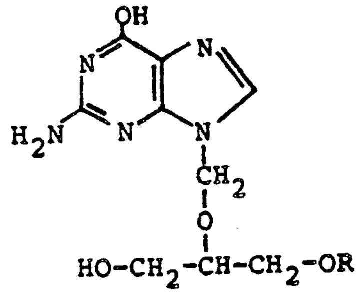

dans laquelle R représente H, un groupe silyle, silyle substitué, alkle en $C_{1-6}$ ou phényle-(alkyle en $C_1$ à $C_6$).

2. Utilisation d'un dérivé de guanine suivant la revendication 1, pour la production d'un médicament destiné au traitement d'infections virales dans des cellules vivantes de mammifères.

**Revendications pour l'Etat contractant: AT**

1. Procédé de préparation d'un dérivé de guanine der formule

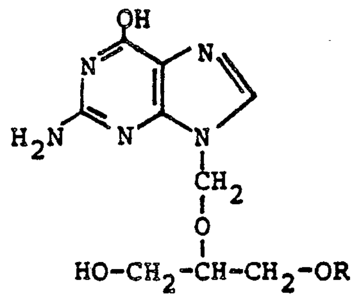

dans laquelle R représente H, un groupe silyle, silyle substitué, alkle en $C_{1-6}$ ou phényle-(alkyle en $C_1$ à $C_6$), qui consiste à faire réagir un guanine, protégée de mainère appropriée, avec un halogénure de 1,3-dihydroxy-2-propoxyméthyle facultativement protégé ou un autre dérivé réactif, et si nécessaire, à éliminer les groupes protecteurs et/ou introduire un groupe R autre que H.

2. Utilisation d'un dérivé de guanine suivant la revendication 1, pour la production d'un médicament destiné au traitement d'infections virales dans des cellules vivantes de mammifères.